## Europäisches Patentamt
## European Patent Office
## Office européen des brevets

(11) Publication number: **0 094 208**
**B1**

(12) **EUROPEAN PATENT SPECIFICATION**

(45) Date of publication of patent specification: **09.12.87**

(51) Int. Cl.⁴: **B 01 J 8/06, C 07 D 301/08**

(21) Application number: **83302539.8**

(22) Date of filing: **05.05.83**

(54) Reactor temperature control systems.

(30) Priority: **07.05.82 US 375795**

(43) Date of publication of application:
**16.11.83 Bulletin 83/46**

(45) Publication of the grant of the patent:
**09.12.87 Bulletin 87/50**

(84) Designated Contracting States:
**DE FR GB IT**

(56) References cited:
**FR-A-2 318 674**
**FR-A-2 356 197**
**US-A-3 271 472**
**US-A-4 236 218**
**US-A-4 249 908**

**CHEMICAL ABSTRACTS, vol. 92, no. 16, 21st April 1980, page 148, no. 131449a, Columbus, Ohio,US; & SU - A - 706 101 (LENINGRAD TECHNOLOGICAL INSTITUTE) (30-12-1979)**

(73) Proprietor: **THE BABCOCK & WILCOX COMPANY**
**1010 Common Street P.O. Box 60035**
**New Orleans Louisiana 70160 (US)**

(72) Inventor: **Agarwal, Suresh C.**
**26011 Lakeshore Boulevard**
**Euclid Ohio 44132 (US)**

(74) Representative: **Cotter, Ivan John et al**
**D. YOUNG & CO. 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to reactor temperature control systems or arrangements, and is related to our earlier European Patent Application No. 83301958.1 (Publication No. EP—A—0 092 924), hereinafter referred to as "the earlier application". Systems embodying the invention may, for example, be used for controlling the temperature of an olefin oxidation reactor by regulating the rate of coolant flow to maintain the reactor temperature within a desired temperature range, during the operation of the reactor as well as during start-up, shut-down and transient operating conditions.

Various techniques and systems are known for controlling chemical reactors.

In addition to the non-anticipating but relevant patents set forth in the earlier application, U.S. Patent No. 3 471 582 to Lupfer discloses an arrangement wherein a desired temperature across an exothermic reactor is maintained by controlling the reactor feed temperature in response to a difference in temperature between the reactant feed and product stream from the reactor until a maximum predetermined product temperature is obtained.

U.S. Patent No. 3 271 472 to Ogle et al discloses apparatus for controlling the operation of a thermal cracking furnace. Since the thermal cracking of hydrocarbons is an endothermic reaction, it is necessary to maintain a maximum possible temperature within the equipment limits. A minimum temperature is not considered or important in Ogle et al.

See also U.S. Patent No. 4 249 907 to Callegas which discloses a selective hydrogeneration process wherein at least one catalyst is utilized. The temperature of feed stream to a catalyst bed is controlled so as to maintain a desired reaction temperature in the catalyst bed.

In an olefin, in particular ethylene, oxide manufacturing process, ethylene and oxygen or air are mixed and fed to an isothermal multitubular reactor. Ethylene is oxidized into ethylene oxide in the presence of a catalyst and carbon dioxide and water are produced as by-products. Reactor temperature control objectives are:

operation at the most economical temperature;

operation within a safe zone;

maximum conversion to ethylene oxide while minimizing by-products;

reduction of consumption of coolant;

avoidance or elimination of unsafe operation; and

reduced operator attention.

Reactor temperature control is of key significance because of the following factors:

1. The most economical temperature for oxidation is one at which occurs the highest conversion to ethylene oxide rather than to by-products.

2. Catalyst selectivity increases as the reaction temperature is lowered while ethylene conversion increases with increasing reactor temperature. Thus, temperature requirements for high selectivity and high conversion are opposed. This results in a narrow temperature range for reactor operation.

3. Increase in reaction temperature produces two effects: (1) the overall rate of ethylene oxidation increases, and (2) catalyst selectivity to ethylene oxide decreases such that relatively more ethylene is converted into carbon dioxide and water. Moreover, heat generation increases due to the fact that more ethylene is oxidized and the overall reaction becomes less selective. Consequently, increase in temperature may result in:

a reactor runaway condition;

catalyst poisoning;

increased coolant demand;

an unsafe operating situation; and/or

increased operator attention.

Thus, neither a temperature rise nor a temperature drop is desirable.

In a state of the art system, reactor temperature control is based on manipulating coolant flow rate. A set point of the system is directly based upon average reactor temperature. Such a control scheme results in almost all of the deficiencies described above.

USSR Patent No. SU—A—706 101 discloses a nitrating reaction system in which the flow of compound to be nitrated and the flow of nitrating agent to the reactor are measured by respective flow transmitters. The flow of coolant to the jacket of the reactor is also measured by a further flow transmitter and the temperature within the reactor is measured by a temperature transmitter. The flow of coolant to the jacket is adjusted by a valve in accordance with the measured reactor temperature and the measured flow rate of the compound to be nitrated.

According to the present invention there is provided a system for controlling the temperature of a reactor for containing a reaction from at least one reactant to at least another product, the reactor having a feed line for the reactant and an effluent line for the product, the system comprising:

a feed flow transmitter connected to the feed line for measuring the flow of reactant to the reactor;

a reactor temperature transmitter connected to the reactor for measuring a temperature of the reactor;

a coolant flow line to the reactor for supplying coolant to the reactor at a coolant flow rate;

coolant flow control means in the coolant line; and

circuit means connected to said feed flow and reactor temperature transmitters and to the coolant flow control means for controlling the flow of coolant to the reactor according to a coolant flow signal;

the system being characterised by:

an effluent flow transmitter connected to the effluent line for measuring the flow of product from the reactor;

a feed temperature transmitter connected to the feed line for sensing the reactant temperature;

an effluent temperature transmitter connected to the effluent line for measuring the product temperature;

a concentration transmitter connected to the effluent line for measuring the concentration of the at least one product in the effluent line;

and in that the circuit means

(i) is connected to said effluent flow transmitter, said feed temperature transmitter, said effluent temperature transmitter and said concentration transmitter,

(ii) is connected to receive quantities proportional to the heat of reaction for at least one reaction in the reactor, specific heats of the reactant and product, and the heat of vaporization of the coolant,

(iii) is operable to obtain values for changes per unit time in feed flow rate, effluent flow rate, feed temperature, reactor temperature, effluent temperature, and concentration of at least one product,

(iv) includes circuit components for multiplying each change per unit time by a characteristic factor to provide partial flow control signals, and

(v) is operable to process arithmetically the partial flow control signals to provide a quantity indicative of required change in coolant flow rate, which quantity is used to change the coolant flow signal.

A preferred embodiment of the present invention described in detail hereinbelow comprises a control arrangement or system which permits operation of an olefin oxidation reactor at the most economical and safe temperature range, with regard to a maximum conversion of the olefin to the desired olefin oxide and a minimization of by-products. The preferred control system is in particular applicable to ethylene oxidation reactors but is also applicable to other exothermic and endothermic reactors.

According to the preferred embodiment of the invention, a system is provided which controls the rate of coolant flow in the chemical reactor according to an algorithm which incorporates various parameters including reactor feed and effluent flow rates, specific heat of reactants and products, reactor and effluent temperatures, coolant heat of evaporation, reactant and product concentration and heat of reactions for various reactions taking place in the reactor. In addition, temperatures are taken at varied locations along the reactor length, for obtaining a maximum and a minimum value for temperatures within the reactor for establishing a desired reactor temperature range. The preferred temperature control system is simple in design, rugged in construction and economical to manufacture.

The preferred control system or arrangement, which operates by controlling the rate of coolant flow, additionally compensates for variations in operating conditions as well as providing proper coolant flow control even during start-up and shut-down phases of the operation.

The invention will now be further described, by way of illustrative and non-limiting example, with reference to the accompanying drawings, in which:

Figure 1 is a schematic representation of a temperature control system embodying the invention in combination with a tubular reactor for containing an olefin oxidation reaction; and

Figure 2 is a block diagram which illustrates an exemplary computer layout for implementing the system.

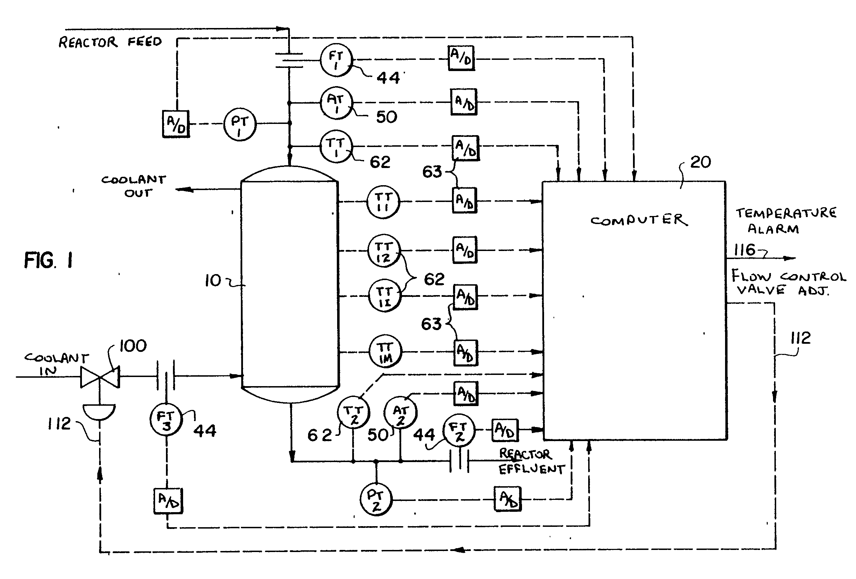

Figure 1 shows a control system or arrangement for controlling the flow of coolant into a tube reactor 10 by controlling the position of a valve 100 in a coolant inlet line. Control of the valve 100 is achieved over a line 112 connected to the output of a computer 20 which receives various inputs carrying signals corresponding to temperatures at various locations in the reactor and in the reactor feed at effluent lines and component concentrations. The computer is programmed with constants which relate to heats of various reactions going on in the reactor 10 and various physical characteristics of the coolant, the reactants and the products of the reaction.

The control system described in the earlier application is based on the following assumptions:

a. Specific heat of reactor feed and effluent streams is assumed to be constant with changes in temperature and steam composition.

b. Heat of reaction is independent of temperature.

The above assumptions, however, while usually valid, do not hold during start-up, shut-down and transient reactor operating conditions. Consequently, coolant flow rate may be less than the desired rate, whereby the reactor will operate at a temperature higher than necessary. The present control system takes these variations into account.

As shown in the earlier application, coolant flow rate is given by the expression

$$Q=\frac{1}{\lambda}[F_2\{y_1\Delta H_1+y_2\Delta H_2+C_{P_1}(T_R-T_O)\}-F_1C_{P_i}(T_R-T_I)] \qquad (1)$$

where:

$Q=$FLow rate of coolant;

$\lambda=$Heat of vaporization for coolant;

$F_1=$Flow rate of feed;

$$C_{P_i}= \sum_{k=1}^{4} C_{P_k} X_k \text{ (for feed)}$$

$C_{P_k}=$specific heat of component k in feed; (k=1 for ethylene, 2 for carbon dioxide, 3 for ethylene oxide and 4 for oxygen);

$X_k=$Concentration of component k in feed;

$T_R=$Reaction temperature;

$T_I=$Feed inlet temperature;

$T_O=$Reactor Exit Stream temperature;

$$C_{P_1}= \sum_{m=1}^{4} C_{P_m} y_m \text{ (for effluent)}$$

$C_{P_m}=$specific heat of component m in effluent; (m=1 for ethylene oxide, m=2 for $CO_2$, m=3 for ethylene, m=4 for water);

$\Delta H_1=$Heat of reaction for oxidation to ethylene oxide;

$\Delta H_2=$Heat of reaction for oxidation to carbon dioxide and water;

$F_2=$Flow rate of reactor effluent;

$y_1=$a first product (ethylene oxide) concentration; and

$y_2=$a second product ($CO_2$) concentration.

Functionally. equation (1) can be written as

$$Q=f[F_1, F_2, T_I, T_R, T_O, y_1, y_2] \qquad (2)$$

Then

$$\frac{dQ}{dt}=[\frac{\partial f}{\partial F_1} \frac{dF_1}{dt}+\frac{\partial f}{\partial F_2} \frac{dF_2}{dt}+\frac{\partial f}{\partial T_I} \frac{dT_I}{st}+\frac{\partial f}{\partial T_R} \frac{dT_R}{dt} \qquad (3)$$

$$+\frac{\partial f}{\partial T_O} \frac{dT_O}{dt}+\frac{\partial f}{\partial y_1} \frac{dy_1}{dt}+\frac{\partial f}{\partial y_2} \frac{dy_2}{dt}]$$

Since dQ/dt is the rate of change of Q with change in t, hence at fixed time intervals

$$\Delta Q=Q(n)-Q(n-1)=\frac{dQ}{dt} \text{ for } t=T,$$

where T=control action interval.

Thus:

$$\Delta Q=[\frac{\partial f}{\partial F_1} \Delta F_1+\frac{\partial f}{\partial F_2} \Delta F_2+\frac{\partial f}{\partial T_I} \Delta T_I+\frac{\partial f}{\partial T_R} \Delta T_R+\frac{\partial f}{\partial T_O}T_O \qquad (4)$$

$$+\frac{\partial f}{\partial y_1} \Delta y_1+\frac{\partial f}{\partial y_2}\Delta y_2]$$

where:

$$\frac{\partial f}{\partial f_1}=-[C_{P_i} (T_R-T_I)]/\lambda \qquad (5)$$

$$\frac{\partial f}{\partial F_2}=y_1\Delta H_1+y_2\Delta H_2+C_{P_1} (T_R-T_O)]/\lambda \qquad (6)$$

$$\frac{\partial f}{\partial T_I} = F_1 C_{P_I}/\lambda \qquad (7)$$

$$\frac{\partial f}{\partial T_R} = (F_2 C_{P_1} - F_1 C_I)/\lambda \qquad (8)$$

$$\frac{\partial f}{\partial T_O} = -F_2 C_{P_1}/\lambda \qquad (9)$$

$$\frac{\partial f}{\partial y_1} = F_2 \Delta H_1/\lambda \qquad (10)$$

$$\frac{\partial f}{\partial y_2} = F_2 \Delta H_2/\lambda \qquad (11)$$

$$C_{P_I} = \sum_{k=1}^{4} C_{P_k} x_k \qquad (12)$$

$$C_{P_1} = \sum_{m=1}^{4} C_{P_m} y_m \qquad (13)$$

and, temperature dependence of specific heat and heat of reactions are given by;

$$\Delta H = \Delta H_0 + \int^T C_p \, dT; \text{ and}$$

$$C_P = a + bT + CT^2$$

A control system embodying the invention can thus be obtained where all measured signals are interfaced to a control computer system by state of the art methods. The total control system is shown in Figure 1.

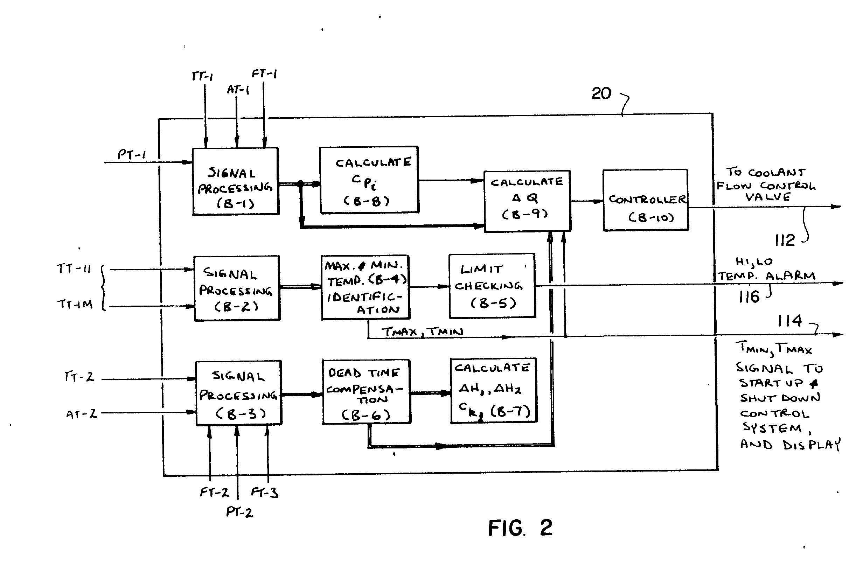

Major steps of the control system are given in Figure 2. The calculations for a block B-9 are those which have been developed above. Calculations in all other blocks are based upon commonly known practices.

The outputs are: coolant flow control valve setting at 112; minimum and maximum temperature signals to start-up and shut-down control systems (not shown here), and to display units at 114; and high/low temperature alarms at 116. Other measured signals can be checked for high and/or low limits and alarmed as per need and operating practices of an individual reactor system.

While the implementation shown herein is through a control computer system, the invention can also easily be implemented by conventional electronic instrumentation and control systems.

Referring once more to the drawings, a plurality of temperature transmitters 62 provide temperature signals to computer 20 for the reactor feed line, the reactor 10 at various longitudinal locations thereof and the reactor effluent line. Each of the analog signals is converted in a corresponding analog to digital converter 63 into a corresponding digital signal which is readable by computer 20. In addition to the temperature transmitters, the system is provided with a plurality of flow transmitters 44 and a plurality of concentration transmitters 50. Flow transmitters 44 provide analog signals which similarly are converted to corresponding digital signals corresponding to the reactor feed and effluent flow rate as well as the coolant flow rate.

Concentration transmitters 50 provide signals corresponding to the concentration of various components in the feed and effluent line.

Each of the transmitters is further identified and correlated with inputs to the computer shown in Fig. 2. Temperature transmitters TT11 to TT1M provide their signals to a signal processing block B-2. The signals are then processed in a block B-4 to determine the maximum and minimum temperatures along the longitudinal length of the reactor. The signal is then supplied over line 14 to an output of computer 20 and also to the block B-9 for achieving calculations as pointed out above. Limit checking is accomplished in a block B-5 for sounding a low or high temperature alarm over line 116.

The transmitters and blocks identified above are all individually available in the art and will not be described in greater detail.

According to the present system, thus, not only is the coolant flow rate Q as set forth in equation (1) obtained, but also the change in coolant flow rate $\Delta Q$. Equation (4) is utilized with each differential factor calculated as shown in equation (5) to equation (11). Simple comparators which relate an initial value with a later value can be utilized in computer 20 to obtain the values of flow rate change, $\Delta F_1$ and $\Delta F_2$, as well as the temperature and concentration change.

## Claims

1. A system for controlling the temperature of a reactor (10) for containing a reaction from at least one reactant to at least another product, the reactor (10) having a feed line for the reactant and an effluent line for the product, the system comprising:

a feed flow transmitter (44) connected to the feed line for measuring the flow $(F_1)$ of reactant to the reactor (10);

a reactor temperature transmitter (62) connected to the reactor for measuring a temperature $(T_R)$ of the reactor;

a coolant flow line to the reactor (10) for supplying coolant to the reactor at a coolant flow rate;

coolant flow control means (100) in the coolant line; and

circuit means connected to said feed flow and reactor temperature transmitters (44, 62) and to the coolant flow control means (100) for controlling the flow of coolant to the reactor (10) according to a coolant flow signal;

the system being characterised by:

an effluent flow transmitter (44) connected to the effluent line for measuring the flow $(F_2)$ of product from the reactor (10);

a feed temperature transmitter (62) connected to the feed line for sensing the reactant temperature $(T_1)$;

an effluent temperature transmitter (62) connected to the effluent line for measuring the product temperature $(T_O)$;

a concentration transmitter (50) connected to the effluent line for measuring the concentration of the at least one product in the effluent line;

and in that the circuit means

(i) is connected to said effluent flow transmitter (44), said feed temperature transmitter (62), said effluent temperature transmitter (62) and said concentration transmitter (50),

(ii) is connected to receive quantities proportional to the heat of reaction for at least one reaction in the reactor (10), specific heats of the reactant and product, and the heat of vaporization of the coolant,

(iii) is operable to obtain values for changes per unit time in feed flow rate $(\Delta F_1)$, effluent flow rate $(\Delta F_2)$, feed temperature $(\Delta T_1)$, reactor temperature $(\Delta T_R)$, effluent temperature $(\Delta T_O)$, and concentration of at least one product $(\Delta y)$,

(iv) includes circuit components for multiplying each change per unit time by a characteristic factor to provide partial flow control signals, and

(v) is operable to process arithmetically the partial flow control signals to provide a quantity $(\Delta Q)$ indicative of required change in coolant flow rate, which quantity $(\Delta Q)$ is used to change the coolant flow signal.

2. A system according to claim 1, wherein the circuit means includes circuit elements for generating factors as follows:

$$\frac{\partial f}{\partial F_1} = -[C_{P_1}(T_R - T_1)]/\lambda$$

$$\frac{\partial f}{\partial F_2} = y_1 \Delta H_1 + y_2 \Delta H_2 + C_{P_1}(T_R - T_O)]/\lambda$$

$$\frac{\partial f}{\partial T_1} = F_1 C_{P_1}/\lambda$$

$$\frac{\partial f}{\partial T_R} = (F_2 C_{P_1} - F_1 C_1)/\lambda$$

$$\frac{\partial f}{\partial T_O} = -F_2 C_{P_1}/\lambda$$

$$\frac{\partial f}{\partial y_1}=F_2\Delta H_1/\lambda$$

$$\frac{\partial f}{\partial y_2}=F_2\Delta H_2/\lambda$$

the coolant flow signal being changed by the quantity $\Delta Q$ in the circuit means which is calculated as follows:

$$\Delta Q=[\frac{\partial f}{\partial F_1}\ \Delta F_1+\ \frac{\partial f}{\partial F_2}\ \Delta F_2+\ \frac{\partial f}{\partial T_1}\ \Delta T_1+\ \frac{\partial f}{\partial T_R}\ \Delta T_R+\ \frac{\partial f}{\partial T_0}T_0$$

$$+\frac{\partial f}{\partial y_1}\ \Delta y_1+\ \frac{\partial f}{\partial y_2}\Delta y_2]$$

wherein:

$\lambda$=coolant heat of vaporization;

$y_1$=a first product concentration;

$\Delta H_1$=heat of reaction of reactant to first product;

$y_2$=a second product concentration;

$\Delta H_2$=heat of reaction of reactant to second product; and

$C_{P_1}$=specific heat of effluent.

3. A system according to claim 1 or claim 2, comprising a plurality of temperature transmitters (62) distributed along the length of the reactor (10), and a minimizing/maximizing circuit connected to the temperature transmitters for obtaining a minimum and a maximum temperature among the temperature transmitters of the reactor.

4. A process for preparing ethylene oxide from ethylene by oxidation using a system according to claim 2, in which ethylene plus oxygen is supplied to a reactor (10) as reactant and ethylene oxide plus carbon dioxide and water are generated as products, $y_1$ being the concentration of ethylene oxide, $y_2$ being the concentration of carbon dioxide, $\Delta H_1$ being the heat of reaction of ethylene plus oxygen to ethylene oxide, and $\Delta H_2$ being the heat of reaction of ethylene plus oxygen to carbon dioxide.

**Patentansprüche**

1. System zur Steuerung der Temperatur eines Reaktors (10) für eine Reaktion von wenigstens einem Reaktionspartner zu wenigstens einem anderen Produkt, wobei der Reaktor (10) eine Beschickungsleitung für den Reaktionspartner und eine Auslaufleitung für das Produkt aufweist, mit:

einem Beschickungsflußmeßwertgeber (44), der mit der Beschikkungsleitung zum Messen des Flusses $(F_1)$ des Reaktionspartners zum Reaktor (10) verbunden ist;

einem Reaktortemperaturmeßwertgeber (62), der mit dem Reaktor zur Messung einer Temperatur $(T_R)$ des Reaktors verbunden ist;

eine Kühlmittelflußleitung zum Reaktor (10) für die Zuführung von Kühlmittel zum Reaktor mit einer Kühlmittelflußrate;

Kühlmittelflußsteuermitteln (100) in der Kühlmittelleitung; und

Schaltungsmitteln, die mit den Meßwertgebern (44, 62) für den Beschickungsfluß und die Reaktortemperatur und mit dem Kühlmittelflußsteuermittel (100) für die Steuerung des Flusses des Kühlmittels zum Reaktor (10) gemäß einem Kühlmittelflußsignal verbunden sind;

wobei das System gekennzeichnet ist durch:

einen Auslaufflußmeßwertgeber (44), der mit der Auslaufleitung für die Messung des Flusses $(F_2)$ des Produktes aus dem Reaktor (10) verbunden ist;

einen Beschickungstemperaturmeßwertgeber (62), der mit der Beschickungsleitung zum Abfühlen der Reaktionspartnertemperatur $(T_1)$ verbunden ist;

einen Auslauftemperaturmeßwertgeber (62), der mit der Auslaufleitung zur Messung der Produkttemperatur $(T_0)$ verbunden ist;

einen Konzentrationsmeßwertgeber (50), der mit der Auslaufleitung für das Messen der Konzentration mindestens des einen Produktes in der Auslaufleitung verbunden ist;

und daß das Schaltkreismittel

(i) mit dem Auslaufflußmeßwertgeber (44), dem Beschickungstemperaturmeßwertgeber (62), dem Auslauftemperaturmeßwertgeber (62) und dem Konzentrationsmeßwertgeber (50) verbunden ist,

(ii) verbunden ist, um Mengen proportional zur Reaktionswärme für mindestens eine Reaktion in dem Reaktor (10), spezifische Wärmen des Reaktionspartners und -produktes und der Verdampfungswärme des Kühlmittels ·aufzunehmen,

(iii) betriebbar ist, um Werte für Änderungen pro Einheitszeit in der Beschickungsflußrate ($\Delta F_1$), Auslaufflußrate ($\Delta F_2$), Beschickungstemperatur ($\Delta T_I$), Reaktionstemperatur ($\Delta T_R$), Auslauftemperatur ($\Delta T_0$) und Konzentration mindestens eines Produktes ($\Delta y$) zu erhalten,

(iv) Schaltkreisbestandteile aufweist zum Multiplizieren jeder Änderung pro Einheitszeit mit einem charakteristischen Faktor, um Teilflußsteuersignale vorzusehen, und

(v) betreibbar ist, um die Teilflußsteuersignale arithmetisch zu verarbeiten, um eine Menge ($\Delta Q$) vorzusehen als Anzeige der erforderlichen Änderung in der Kühlmittelflußrate, wobei die Menge ($\Delta Q$) verwendet wird, um das Kühlmittelflußsignal zu verändern.

2. System nach Anspruch 1, wobei das Schaltkreismittel Schaltkreiselemente aufweist zur Erzeugung von Faktoren wie folgt:

$$\frac{\partial f}{\partial F_1} = -[C_{P_I}(T_R - T_I)]/\lambda$$

$$\frac{\partial f}{\partial F_2} = y_1\Delta H_1 + y_2\Delta H_2 + C_{P_1}(T_R - T_0)]/\lambda$$

$$\frac{\partial f}{\partial T_I} = F_1 C_{P_I}/\lambda$$

$$\frac{\partial f}{\partial T_R} = (F_2 C_{P_1} - F_1 C_I)/\lambda$$

$$\frac{\partial f}{\partial T_0} = -F_2 C_{P_1}/\lambda$$

$$\frac{\partial f}{\partial y_1} = F_2\Delta H_1/\lambda$$

$$\frac{\partial f}{\partial y_2} = F_2\Delta H_2/\lambda$$

wobei das Kühlmittelflußsignal durch die Menge $\Delta Q$ im Schaltkreismittel geändert wird, welche sich wie folgt berechnet:

$$\Delta Q = [\frac{\partial f}{\partial F_1}\Delta F_1 + \frac{\partial f}{\partial F_2}\Delta F_2 + \frac{\partial f}{\partial T_I}\Delta T_I + \frac{\partial f}{\partial T_R}\Delta T_R$$

$$+\frac{\partial f}{\partial T_0}T_0 + \frac{\partial f}{\partial y_1}\Delta y_1 + \frac{\partial f}{\partial y_2}\Delta y_2]$$

wobei:

$\lambda$=Kühlmittelverdampfungswärme;
$y_1$=Konzentration eines ersten Produktes;
$\Delta H_1$=Reaktionswärme des Reaktionspartners zum ersten Produkt;
$y_2$=Konzentration eines zweiten Produktes;
$\Delta H_2$=Reaktionswärme des Reaktionspartners zum zweiten Produkt; und
$C_{P_1}$=spezifische Wärme des Auslaufes.

3. System nach Anspruch 1 oder Anspruch 2, mit einer Vielzahl von Temperaturmeßwertgebern (62), die längs des Reaktors (10) verteilt sind, und einem Minimierungs/Maximierungsschaltkreis, der mit den Temperaturmeßwertgebern verbunden ist, um eine minimale und eine maximale Temperatur unter den Temperaturmeßwertgebern des Reaktors zu erhalten.

4. Verfahren zur Herstellung von Ethylenoxid aus Ethylen durch Oxidation unter Verwendung eines Systems nach Anspruch 2, bei welchem Ethylen plus Sauerstoff einem Reaktor (10) als Reaktionspartner zugeführt wird und Ethylenoxid plus Kohlendioxid und Wasser als Produkte erzeugt werden, wobei $y_1$ die Konzentration des Ethylenoxids, $y_2$ die Konzentration von Kohlendioxid ist, $\Delta H_1$ die Reaktionswärme der Reaktion von Ethylen plus Sauerstoff zu Ethylenoxid ist und $\Delta H_2$ die Reaktionswärme der Reaktion von Ethylen plus Sauerstoff zu Kohlendioxid ist.

**Revendications**

1. Un système pour commander la température d'un réacteur (10) destiné à contenir une réaction faisant passer d'au moins une substance réactive à au moins un autre produit, le réacteur (10) comportant un conduit d'alimentation pour la substance réactive et un conduit d'effluent pour le produit, le système comprenant:

un émetteur de débit d'alimentation (44) connecté au conduit d'alimentation pour mesurer le débit ($F_1$) de substance réactive entrant dans le réacteur (10);

un émetteur de température de réacteur (62) connecté au réacteur pour mesurer une température ($T_R$) du réacteur;

un conduit d'écoulement de fluide de refroidissement dirigé vers le réacteur (10), destiné à fournir un fluide de refroidissement au réacteur, avec un débit de fluide de refroidissement;

des moyens de commande de débit de fluide de refroidissement (100) dans le conduit de fluide de refroidissement; et

un circuit connecté aux émetteurs de débit d'alimentation et de température de réacteur (44, 62), ainsi qu'aux moyens de commande de débit de fluide de refroidissement (100), pour commander le débit de fluide de refroidissement vers le réacteur (10) conformément à un signal de débit de fluide de refroidissement;

le système étant caractérisé par:

un émetteur de débit d'effluent (44) connecté au conduit d'effluent pour mesurer le débit ($F_2$) du produit qui sort du réacteur (10);

un émetteur de température d'alimentation (62) connecté au conduit d'alimentation pour détecter la température de la substance réactive ($T_1$);

un émetteur de température d'effluent (62) connecté au conduit d'effluent pour mesurer la température du produit ($T_O$);

un émetteur de concentration (50) connecté au conduit d'effluent pour mesurer la concentration du produit ou des produits dans le conduit d'effluent;

et en ce que le circuit

(i) est connecté à l'émetteur de débit d'effluent (44), à l'émetteur de température d'alimentation (62), à l'émetteur de température d'effluent (62) et à l'émetteur de concentration (50),

(ii) est connecté de façon à recevoir des quantités proportionnelles à la chaleur de réaction pour au moins une réaction se produisant dans le réacteur (10), aux chaleurs spécifiques de la substance réactive et du produit, et à la chaleur de vaporisation du fluide de refroidissement,

(iii) est capable de fournir des valeurs pour des variations par unité de temps du débit d'alimentation ($\Delta F_1$), du débit d'effluent ($\Delta F_2$), de la température d'alimentation ($\Delta T_1$), de la température du réacteur ($\Delta T_R$), de la température d'effluent ($\Delta T_O$), et de la concentration d'au moins un produit ($\Delta y$),

(iv) comprend des composants de circuit destinés à multiplier par un facteur caractéristique chaque variation par unité de temps, pour fournir des signaux de commande de débit partiels, et

(v) est capable de traiter arithmétiquement les signaux de commande de débit partiels pour fournir une quantité ($\Delta Q$) représentative de la variation exigée du débit de fluide de refroidissement, cette quantité ($\Delta Q$) étant utilisée pour changer le signal de débit de fluide de refroidissement.

2. Un système selon la revendication 1, dans lequel le circuit comprend des éléments de circuit destinés à générer des facteurs, de la manière suivante:

$$\frac{\partial f}{\partial F_1} = -[C_{P_I}(T_T - T_I)]/\lambda$$

$$\frac{\partial f}{\partial F_2} = [y_1 \Delta H_1 + y_2 \Delta H_2 + C_{P_1}(T_R - T_O)]/\lambda$$

$$\frac{\partial f}{\partial T_I} = F_1 C_{P_I}/\lambda$$

$$\frac{\partial f}{\partial T_R}=(F_2 C_{P_1}-F_1 C_1)/\lambda$$

$$\frac{\partial f}{\partial T_O}=-F_2 C_{P_1}/\lambda$$

$$\frac{\partial f}{\partial y_2}=F_2 \Delta H_2/\lambda$$

le signal de débit de fluide de refroidissement étant changé dans le circuit de la quantité $\Delta Q$ qui est calculée de la manière suivante:

$$\Delta Q=[\frac{\partial f}{\partial F_1}\Delta F_1+\frac{\partial f}{\partial F_2}\Delta F_2+\frac{\partial f}{\partial T_1}\Delta T_1+\frac{\partial f}{\partial T_R}\Delta T_R+\frac{\partial f}{\partial T_O}T_O$$

$$+\frac{\partial f}{\partial y_1}\Delta y_1+\frac{\partial f}{\partial y_2}\Delta y_2]$$

avec les notations suivantes:

$\lambda$=chaleur de vaporisation du fluide de refroidissement;
$y_1$=concentration d'un premier produit;
$\Delta H_1$=chaleur de réaction pour le passage de la substance réactive au premier produit;
$y_2$=concentration d'un second produit;
$\Delta H_2$=chaleur de réaction pour le passage de la substance réactive au second produit; et
$C_{P_1}$=chaleur spécifique de l'effluent.

3. Un système selon la revendication 1 ou la revendication 2, comprenant un ensemble d'émetteurs de température (62) répartis sur la longueur du réacteur (10), et un circuit de détermination de minimum/maximum connecté aux émetteurs de température pour obtenir une température minimale et une température maximale à partir des émetteurs de température du réacteur.

4. Un procédé pour préparer de l'oxyde d'éthylène à partir de l'éthylène, par oxydation, en utilisant un système selon la revendication 2, dans lequel on fournit de l'éthylène plus de l'oxygène à un réacteur (10) en tant que substance réactive, et de l'oxyde d'éthylène plus du dioxyde de carbone et de l'eau sont générés en tant que produits, et dans lequel $y_1$ est la concentration d'oxyde d'éthylène, $y_2$ est la concentration de dioxyde de carbone, $\Delta H_1$ est la chaleur de réaction de l'éthylène et de l'oxygène pour donner de l'oxyde d'éthylène, et $\Delta H_2$ est la chaleur de réaction de l'éthylène et de l'oxygène pour donner du dioxyde de carbone.

FIG. 1

REACTOR FEED

COOLANT
OUT

COOLANT
IN

COMPUTER

20

TEMPERATURE
ALARM

116

FLOW CONTROL
VALVE ADJ.

112

REACTOR
EFFLUENT

100

102

0 094 208

FIG. 2